(19) Europäisches Patentamt
European Patent Office
Office européen des brevets

(11) Publication number: **0 051 071**
**B1**

(12) **EUROPEAN PATENT SPECIFICATION**

(45) Date of publication of patent specification: **20.02.85**

(51) Int. Cl.$^4$: **C 07 H 11/04,** C 12 P 21/02, A 61 K 37/02, C 12 N 1/20

(21) Application number: **79901543.3**

(22) Date of filing: **15.11.79**

(86) International application number: **PCT/JP79/00295**

(87) International publication number: **WO 80/01069 29.05.80 Gazette 80/12**

(54) **TRYPTOPHANE DERIVATIVE AND PROCESS FOR PREPARING SAME.**

(30) Priority: **17.11.78 JP 142081/78**

(43) Date of publication of application: **12.05.82 Bulletin 82/19**

(45) Publication of the grant of the patent: **20.02.85 Bulletin 85/08**

(84) Designated Contracting States: **CH DE FR GB**

(56) References cited:

**Chemical Abstracts, vol. 80, no. 5, (1974-02-04) (Columbus, Ohio, U.S.A.), H. Umezawa et al. "Thermolysin inhibitor produced by actinomycetes: Phosphoramidon", see page 316**

(73) Proprietor: **AJINOMOTO CO., INC.**
**5-8, Kyobashi 1-chome, Chuo-ku**
**Tokyo 104 (JP)**

(72) Inventor: **MURAO,Sawao**
**8-12, Horiagemidorimachi 2-chome**
**Sakai-shi, Osaka 593 (JP)**
Inventor: **FUKUHARA, Kenichi**
**1230-231, Kamigo-cho Totsuka-ku**
**Yokohama-shi,Kanagawa 247 (JP)**

(74) Representative: **Jones, Michael Raymond**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 051 071 B1**

**Description**

This invention relates to a novel tryptophan derivative having an inhibitory activity against metallo proteinases; a process for biochemically producing the tryptophan derivative; and novel microorganisms suitable for use in that process. The novel metallo proteinase inhibitor is useful in medicine, for example as an agent for treating *Pseudomonas aeruginosa* infectious disease and as an agent for regulating growth of various microorganisms. The microorganisms suitable for use in producing the novel tryptophan derivative belong to the species *Streptomyces mozunensis,* which is a new species of the genus *Streptomyces.*

Proteinase inhibitors are useful in the medical field, and some of them are put to practical use. Particularly, it is thought that a metallo proteinase inhibitor can be used as an agent for treating *Pseudomonas aeruginosa* infectious disease, because it is known that the metallo proteinase of *Pseudomonas aeruginosa* plays an important part in *Pseudomonas aeruginosa* infectious disease.

Furthermore, in the field of biochemistry, a material having inhibitory activity against a metallo proteinase produced by a microorganism, is useful; for example, it is expected that the material can be used for regulating growth and metabolism of that microorganism.

Accordingly, in the field of medicine and biochemistry, the provision of a material which has an inhibitory effect against metallo proteinase produced by *Pseudomonas aeruginosa* is important.

Phosphoramidon (as disclosed in The Journal of Antibiotics *26*, [10], 621 (1973) and S-MPI (as disclosed in Agric. Biol. Chem., *42,* [4], 899 (1978)) are known as metallo proteinase inhibitors produced by the genus *Streptomyces.*

According to one aspect of the present invention there is provided, as a novel compound, N-(6-deoxy-L-talosyloxy hydroxyphosphinyl)-L-leucyl-L-tryptophan having the following structural formula:

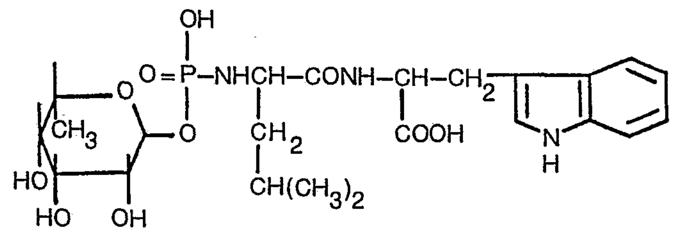

wherein hereinafter may be referred to as the novel tryptophan derivative in which one or more hydroxy group in the sugar moiety is optionally protected by acylation, or a salt of the optionally protected compound, or an ester involving one or both of the said moieties of the optionally protected compound.

The term "optionally protected compound" as used herein is intended to cover both the compound in the unprotected condition as well as the compound in the protected condition.

Another aspect of the present invention provides a process for producing the novel tryptophan derivative, which comprises culturing a microorganism belonging to the species *Streptomyces mozunensis* and capable of producing the novel tryptophan derivative, which microorganism may be referred to as the novel tryptophan derivative producing microorganism; producing the novel tryptophan derivative in the culture medium; and recovering it.

As indicated above, the novel tryptophan derivative may be in the salt form or ester form. Additionally or alternatively, the hydroxyl group in the sugar moiety of the compound may be acylated. For instance, in the salt or ester form, the phosphoric acid moiety and/or carboxyl group may be modified. Examples of the salt are metal salts such as sodium, potassium, rubidium, cesium or calcium salt, and ammonium salt. Examples of the ester are alkyl, aryl and aralkyl esters such as methyl, ethyl, phenyl and benzyl esters.

As the acylating group for the hydroxyl group(s) of the sugar moiety, there can be used one commonly used as a protecting group for hydroxyl groups of sugar moieties, for example an acetyl or benzoyl group.

The novel tryptophan derivative can be produced by culturing the novel tryptophan derivative producing microorganism and recovering the tryptophan derivative produced in the culture medium.

A further aspect of the present invention is a novel microorganic strain referred to as *Streptomyces mozunensis* AJ 9406, FERM-P 4589, NRRL 12054. The AJ number is an Ajinomoto internal reference number, and the two other numbers are the accession numbers of, respectively, the Fermentation Research Institute, Agency of Industrial Science and Technology, Ministry of Internal Trade and Industry, Yatabe-cho-Higashi, Tsukuba-Gun, Ibaragi-ken, Japan, (where the microorganism was deposited on the 22nd November 1978) and the Agricultural Research Culture Collection, Northern Regional Research Center, Peoria, Illinois, U.S.A. (where the microorganism was deposited on the 10th October 1979). Full details of the novel strain appear later in this description.

As the liquid medium used in the culture of the microorganism, one in common use for culturing ordinary microorganisms may be used. There should also be present a carbon source which can be assimilated by the novel tryptophan derivative producing microorganism, for example a carbohydrate such as glucose galactose, maltose, starch or hydrolyzed starch, or an alcohol such as glycerol; a nitrogen source, for example ammonium salts such as ammonium sulphate and ammonium chloride,

amino acids, peptone, meat extract or hydrolyzed soy bean; inorganic salts, and material containing minor nutrients, if necessary.

The microorganism may be cultured, conveniently at a pH of from 5.0 to 9.0, and at a temperature of from 15 to 40°C under an aerobic condition such as shaking and aeration with stirring.

The novel tryptophan derivative is produced in the culture medium of the novel tryptophan derivative producing microorganism and in the microorganism. It is produced mostly in the medium. The novel tryptophan derivative is isolated from the cultured medium of the microorganism by a combination of appropriate methods of separation and purification, such as extraction with butanol, dialysis, adsorption by adsorbent such as activated carbon, and chromatographic techniques such as ion exchange, adsorption, partition and gel filtration chromatography.

There follows an example for the production of the novel tryptophan derivative:

A medium which contained peptone, meat extract and sodium chloride in a ratio of 10 grams of peptone, 10 grams of meat extract and 3 grams of sodium chloride per 1 l of water, was adjusted to pH 7.0. Ten 40 ml aliquots of such medium were poured into ten 500 ml Sakaguchi flasks, respectively.

The flasks were heated for sterilization at 120°C for 10 minutes. In respect of each flask, one loopful inoculum of *Streptomyces mozunensis* AJ 9406 FERM-P 4589, NRRL 12054 was transferred from a slant culture to the culture medium. The resulting medium was shaken at 30°C for 24 hours (120 times/minute).

400 Millilitres of the cultured medium obtained above were transferred to a 20 l jar fermentor made of stainless steel which contained 15 l of sterilized medium, which was adjusted to pH 7.0 and had a composition of glycerol 10 g, peptone 40 g, potassium phosphate ($K_2HPO_4$) 1 g, sodium chloride 1 g, magnesium sulphate 7 hydrate 0.5 g, ferrous sulphate 7 hydrate 0.01 g, cupric sulphate 5 hydrate 1 mg, zinc sulphate 7 hydrate 1 mg, and manganese sulphate 6 hydrate 1 mg, per 1 l of water. The obtained mixture was stirred (at 300 rpm) for main fermentation at 30°C with sterilized air (20 l/minute). After 23 hours' fermentation, the microorganism was removed by centrifuging from the culture medium (pH value: about 8.5) and thereby a supernatant liquid (14 l) was obtained. The inhibitory activity of 50 $\mu$l of that supernatant against the metallo proteinase of *Pseudomonas aeruginosa* was 50%, according to the method of measuring inhibition ratio of enzyme activity mentioned hereinbelow.

To this supernatant (14 l), activated carbon (produced by Wako Pure Chemical Industries Co., Inc.) (700 g) was added and the resulting mixture was stirred for 30 minutes. The carbon was separated by filtration and washed with 3 l of pure water 3 times, and then washed with 15 l of 90% methanol containing ammonia (0.1*N* solution). The thus obtained washings were mixed and then concentrated under reduced pressure to 400 ml. The thus concentrated solution was adjusted to pH 2 at 4°C by adding 3 normal hydrochloric acid. Organic material was extracted with *n*-butanol (400 ml). The thus obtained butanol layer was adjusted to pH 7 at 4°C by adding 3 normal sodium hydroxide solution, and the butanolic mixture was extracted with water (400 ml). The aqueous layer was evaporated to dryness under vacuum and the residue was dissolved in 200 ml of 1 normal acetic acid.

The thus obtained solution was introduced into a column (4.5 cm×25 cm) of DEAE-Sephadex® A-25 (produced by Pharmacia Co., Inc.) which had been well washed with 1 normal acetic acid at 4°C, and then the column was washed with 2.8 l of 1 normal acetic acid. Linear gradient elution from 4 l of 1 normal acetic acid to 4 l of 1 normal acetic acid containing sodium chloride (0.5 M) was carried out. The thus fractionated eluent was collected by a fraction collector and thereby fractions having inhibitory activity against the metallo proteinase of *Pseudomonas aeruginosa* were collected.

These collected fractions were mixed and poured into a column (3.5 cm×15 cm) of activated carbon for chromatography (produced by Wako Pure Chem. Ind. Co., Inc.) which had been well washed with conc. hydrochloric acid, methanol, and water successively. The column was well washed with water until eluent from the column showed negative silver nitrate-reaction. An elution with 85% methanol containing ammonia (pH 10.5) was carried out.

The fractions containing the desired product were combined, concentrated to 3 ml under vacuum, and applied to a column (2.5 cm×40 cm) of Sephadex® G-10 (produced by Pharmacia Co., Inc.) which had been previously well washed with water. An elution with 200 ml of water was carried out. The desired product was eluted in about the first 135 ml of eluate. The fraction containing the desired product was freeze-dried to give a white powder (500 mg).

This white powder had the following properties, and therefrom it was determined as N-(6-deoxy-L-talosyl-oxyhydroxyphosphinyl)-L-leucyl-L-tryptophan.

1. Elemental Analysis (di-cyclohexylammonium salt): Found C 56.60%, H 8.21%, N 9.31%; Calcd. for $C_{23}H_{34}N_3O_{10}P \cdot (C_6H_{13}N)_2$ C 56.67%, H 8.15%, N 9.44%.
2. Molecular Weight (Method according to Mass Spectrometry): 543.
3. Melting point (di-cyclohexylammonium salt): 153~156°C (dec.).
4. Specific Rotation: $[\alpha]_D^{21}$=−25.6° (di-cyclohexyl ammonium salt: C=0.5, $H_2O$).
5. IR (KBr) Spectra: Figure 1
   $\nu_{max}$ ($cm^{-1}$): 3400~3100 (OH, NH), 2950 (CH)~1640 (Amide I), 1590 (Phenyl), 1520 (Amide II), 1400 (CH), 1200 (P=O), ~1070 (C—O), 740 (Phenyl).

6. Colour Reaction: Positive to Rydon-Smith, Ehrlich, diphenylamine-aniline and molybdate-perchloric acid reagents; negative to ninhydrin reagent.
7. Constituting Amino Acid: Leucine and Tryptophan (The material hydrolyzed with 6 normal hydrochloric acid or 4 normal sodium hydroxide was analyzed according to Automatic Amino Acid Analyzer for determination of Amino Acid.)
8. Constituting Sugar: 6-deoxy-L-talose (Experiment: A sample was stood overnight in 0.5 normal hydrochloric acid at room temperature for hydrolysis. The solution obtained by hydrolysis was successively passed through a column packed with active carbon for chromatography (produced by Pure Chem. Ind. Co., Inc), Dowex® 50 [$H^+$], (produced by Dow Chemical Co., Inc.), and Amberlite® IRA-402 [$OH^-$] (produced by Rohm & Haas Co., Inc.). The eluate was concentrated under reduced pressure to isolate a sugar. The sugar was identified as 6-deoxy-L-talose, from the value of specific rotation, thin layer chromatography, gas chromatography and high performance liquid chromatography.)
9. Thin Layer Chromatography: ① $R_f$=0.41 (single spot, thin layer plate of Kieselgel 60 produced by E. Merck Co., Inc.; Developing solvent, n-butanol-acetic acid-water (4:1:1), Colour-producing reagent: 10% sulphuric acid) ② $R_f$=0.49 (single spot; developing solvent, n-butanol-pyridine-acetic acid-water (15:10:3:12); other conditions, mentioned above.)
10. UV Spectra: $\gamma_{max}^{H_2}(\varepsilon)$~280 (5,700).
11. NMR Spectra:
    (1) 'H—NMR (90 MHz, in $D_2O$) was shown in Table 1.
    Internal Standard: 3-(trimethylsilyl)-propane sulphonic acid sodium salt
    (2) $^{13}C$—NMR (25.2 MHz, in $D_2O$) was shown in Table 2.
    Internal Standard: Dioxan
    Chemical Shift; lower magnetic field than that of dioxan: +,
    higher magnetic field than that of dioxan: —.

TABLE 1

| Chemical shift (δ) | | Number of proton, assignment of signals |
|---|---|---|
| 0.8 | ppm | 6H, hydrogen in $\delta$, $\delta'$ position of leucine |
| 1.2 | " | 3H, $\alpha$, J=6.5 Hz, hydrogen of methyl group in sugar |
| 1.0~1.7 | " | 3H, m hydrogen in $\beta$, $\gamma$ position of leucine |
| 3.1~3.8 | " | 6H, m hydrogen in 2, 3 and 4 position of sugar, hydrogen in $\beta$ position of tryptophan, hydrogen in $\alpha$-position of leucine |
| 3.95 | " | 1H, q, J=6.5 Hz, hydrogen in 5 position of sugar |
| 4.7 | " | 1H hydrogen in $\alpha$ position of tryptophan |
| 5.35 | " | 1H, dd, Jp · CH=8 Hz, $J_{1,2}$=1 Hz, hydrogen in 1 position of sugar |
| 7.0~7.8 | " | 5H, m, hydrogen in indole ring of tryptophan |

TABLE 2

| Chemical shift (δ) | Assignment of signal |
|---|---|
| 111.2 ppm<br>110.2 | Carbonyl carbon in tryptophan and leucine |
| 69.3<br>60.8<br>57.6<br>55.0<br>52.4<br>52.2<br>45.1<br>43.3 | Carbon in indole ring of tryptophan |
| 29.3 | d, J=5.4 Hz (*P*—O—*C*), Carbon in 1 position of sugar |
| 5.4 | Carbon in 3 or 4 position of sugar |
| 3.7 | d, J=7.9 Hz (*P*—O—C—*C*), carbon in 2 position of sugar |
| 1.5 | Carbon in 3 or 4 position of sugar |
| −1.5 | Carbon in 5 position of sugar |
| −11.1<br>−11.9 | Carbon in $\alpha$ position of leucine or tryptophan |
| −23.6 | d, J=5.6 Hz (*P*—N—C—*C*), carbon in $\beta$ position of leucine |
| −38.9 | Carbon in $\beta$ position of tryptophan |
| −42.6 | Carbon in $\gamma$ position of leucine |
| −44.0<br>−45.6 | Carbon in $\delta$, $\delta'$ positon of leucine |
| −50.8 | Carbon in 6 position of sugar |

12. Solubility: Soluble in water, methanol and ethanol.
    Insoluble in benzene, hexan, ether and chloroform.
13. Colour: White.
14. Action on various proteinases:
    The novel tryptophan derivative has strong inhibiting activity against various metallo proteinase. Particularly, it strongly inhibits metallo proteinase produced by *Pseudomonas aeruginosa,* and thermolysin which is metallo proteinase produced by the genus Bacillus. It also inhibits metallo proteinase produced by *Aspergillus oryzae;* however, it does not inhibit any of pepsin, trypsin, chymotrypsin, subtilis and papain.
    Amounts of the tryptophan derivative necessary for inhibiting 50% of the enzyme activity are as follows:

| | | |
|---|---|---|
| (1) | Thermolysin | 0.35 μg |
| (2) | Metallo proteinase produced by *Pseudomonas aeruginosa* | 4 μg |

[Method for measuring inhibitory activity against various proteinases]
A mixture of 0.25 ml of sample solution of 0.05 M phosphate buffer at pH 7.5 and 0.25 ml of thermolysin solution (20 μg/ml in the same buffer) was incubated for 10 minutes at 37°C. The mixture was supplemented with 1.5 ml of 1.33% Hammarsten casein solution in 0.1 M phosphate buffer (pH 7.0) and incubated for 10 minutes at 37°C. By the addition of 2 ml of 0.44 M

trichloroacetic acid the reaction was stopped. After standing for 30 minutes at 37°C, the reaction mixture was filtered. To 1.0 ml of the filtrate 5 ml of 0.55 M $Na_2CO_3$ and 1 ml of 2 times diluted Folin-Ciocaltean reagent were added. After standing for 60 minutes at 37°C, the absorbance (Is) was measured at 660 m$\mu$. The blank value (Ib) was taken by carrying out the reaction using 0.25 ml of 0.05 M phosphate buffer (pH 7.5) instead of thermolysin. The control values (Es, Eb) were taken by carrying out such two reactions using 0.25 ml of 0.05 M phosphate buffer (pH 7.5) instead of the sample solution, respectively. Inhibition rate against enzyme activity is calculated by the following equation:

$$\left(1-\frac{Is-Ib}{Es-Eb}\right)\times 100(\%)$$

Now, inhibitory activity against metallo proteinase produced by *Pseudomonas aeruginosa* can be measured in the same manner as above wherein amounts of enzyme used is established so as to have following value.
A value of (Es—Eb) according to method for measuring inhibitory activity is 0.6 wherein cell length used for the measurement of absorbance is 1 cm.

15. Stability: Stable in weak acidic, neutral, or alkaline aqueous solution; Unstable in acidic aqueous solution (Activity is lost completely on standing for 24 hours at pH 1.)

The taxonomic criteria and methods, according to the reports of the International Streptomyces Project (ISP; E. B. Shirling and D. Gottlieb Int. J. Syst. Bacterial., *16*, 313, 1966), were employed and the identification was made by the description of Bergey's Manual of Determinative Bacteriology 8th ed. ("Bergey's Manual of Determinative Bacteriology", ed. by R. E. Buchanan and N. E. Gibbons. The Williams & Wilkins Co., Baltimore, 1974), and ISP reports. The morphological, physiological and cultural properties of the strain AJ-9406, FERM-P 4589, NRRL 12054 are as follows:

a) Morpholigical characteristics are as follows:

| | |
|---|---|
| Spore-bearing hyphae | Simple branching (width: 0.5~1.0 $\mu$m), Retinaculi aperti, Spirales, and Rectiflexibiles |
| Surface structure of spores | Smooth |
| Size of spores | 0.7~1.2x0.9~1.6 $\mu$m |
| Number of spores | 10~30 |
| Flagellated spores | Not detected |
| Globular sporangra | Not detected |
| Sporophore | Formed from aerial mycelium |
| Cell wall type | Type 1 |

b) Cultural characteristics

Observation of growing state in following each medium are all obedient to Method Manual, 1964 of the ISP, and the description is treated the same as example for description in the ISP.

Cultural Characteristics of Strain AJ-9406 on ISP Media after Incubation for 3 weeks at 27~30°C are as follows:

(1) On sucrose-nitrate agar
AM: gray, very thin powdery
VG: colourless, small colonies, scant growth
SP: none

(2) On glucose-asparagine agar
AM: gray, slightly olive, powdery
VG: pale yellow, small colonies
SP: none or trace

(3) On glycerine-asparagine agar (Isp. Med. No. 5)
AM: gray, powdery
VG: colourless-pale yellow, small colonies
SP: pale yellowish brown

(4) On starch-inorganic salts agar (ISP Med. No. 4)
AM: gray, powder
VG: pale yellow-reddish brown, small colonies
SP: none

(5) On tyrosine agar (ISP Med. No. 7)
AM: gray, powdery
VG: yellowish brown-slightly reddish brown, small colonies, slightly wrinkled
SP: light brown-reddish brown

(6) On nutrient agar
AM: none
VG: pale yellow-cream, small colonies
SP: none

(7) On yeast-malt extract agar (ISP Med. No. 2)
AM: gray, powdery
VG: yellowish brown, small colonies-slightly wrinkled
SP: faint brown

(8) On oatmeal agar (ISP Med. No. 3)
AM: gray, very thin powdery
VG: colourless, small colonies, very scant growth
SP: none

(9) On peptone-yeast iron agar
AM: none
VG: pale yellow-cream, small colonies
SP: none

(10) On milk
AM: none
VG: white-pale yellow, growth on surface ring
SP: none or trace

(11) On glucose-peptone gelatin stab
AM: none
VG: pale yellow-cream
SP: none

AM: mass colour and morphology of aerial mycelium.
VG: growth of colony and colour of substrate mycelium.
SP: soluble pigment.

c) Physiological characteristics of strain AJ-9406 are as follows:

| | |
|---|---|
| (1) Hydrolysis of starch | Positive (weak) |
| (2) Liquefaction of gelatin | Positve (slow) |
| (3) Melanoid pigment formation | |
| On peptone-yeast iron agar | Negative |
| On tyrosine agar | Negative |
| On tryptone-yeast extract broth | Negative |
| (4) Coagulation of milk | Positive |
| Peptonization of milk | Negative |
| (5) Cell wall type | Type 1 |

(6) Growth temperature 15—40°C, optimum 30°C

d) Utilization of carbon sources

| Carbon source | Utilization* |
|---|---|
| L-Arabinose | — |
| D-Xylose | — |
| D-Fructose | — |
| D-Glucose | + |
| D-Galactose | + |
| D-Mannose | — |
| L-Rhamnose | — |
| Maltose | + |
| Lactose | — |
| Sucrose | — |
| Raffinose | — |
| Inositol | — |
| D-Mannitol | — |
| Cellulose | — |
| Starch | + |
| Glycerol | + |
| Salicin | — |

Pridham-Gottlieb basal medium
*+, good utilization; —, poor or no utilization

This strain (AJ 9406) is as follows:

(1) Substrate mycelium
Not segmented

(2) Aerial mycelium
Simple branching, Retinaculi aperti, Spirales, and Rectiflexibles
Not formed on vegetative mycelium
Spore formation
Number of spores

(3) Special Breeding Organ such as Flagellated Spores and Globular Sporangia
Not detected

as shown in the morphological characteristics described above.

These results suggest that the strain AJ-9406 should be allocated to the genus *Streptomyces.*

From results of the above characteristics observed, properties of this microorganism (AJ-9406) are summarized as follows:

(1) Spore-bearing hyphae
Simple branching, Retinaculi aperti, Spirales, and Rectiflexibles

(2) Surface Structure of Spores
Smooth

(3) Colour of aerial mycelium
Gray

(4) Colour of substrate mycelium
Pale yellow grown→reddish
Reddish brown on starch-inorganic salts agar

(5) Distinctive colour pigment
Not produced

(6) Sugar utilization
Sugar other than glucose and galactose: poor or no utilization

(7) Melanoid pigment formation
Negative

According to the primary keys noted in Bergey's Manual, strain AJ-9406 belongs to the gray series of *Streptomyces.*

Comparing these microbiological characteristics with those of known species which was described in "The Actinomycetes (Vol II.)" written by S. A. Waksman and the "Cooperative description of Type Culture of Streptomyces II, III, IV, V" written by E. B. Shirling and D. Gottlieb (Int. J. Syst. Bact., *18*, 69—189, 279—392, *19*, 391—512, *22*, 265—394) and the results were summarized in Table 3.

As shown in Table 3, microbiological characteristics of strain AJ-9406 were different from those of the known species. For example, *Actinomyces cyanocolor, Streptomyces nogalater, St. griseoaurantiacus* and *St. Viridifaciens* produce the distinctive soluble pigment, while strain AJ-9406 does not produce it. Moreover, the utilization pattern of carbon sources of these four species are different from that of strain AJ-9406.

*St. recifensis, St. chibaensis* and *St. corchorusii* do not produce the distinctive pigment in the substrate mycellium on starch-inorganic salts agar, while strain AJ-9406 produces it (reddish-brown). Moreover, the utilization patterns of carbon sources of those three species are different from that of strain AJ-9406.

*St. carnosus* produces the distinctive soluble pigment (pale pink or yellow) and utilizes rhamnose, while strain AJ-9406 neither produces the pigment nor utilizes rhamnose.

TABLE 3

| Characteristics / Strain | AJ-9406 | *Actinomyces cyanocolar* | *Streptomyces nogalater* | *St. griseo-aurantiacus* |
|---|---|---|---|---|
| Structure of[a] aerial mycelium | RA, S, RF | S, RA, RF | S, RF, RA | S (RA) |
| Surface structure of spore | Smooth | Smooth | Smooth | Smooth |
| Colour of aerial mycelium | Gray | Gray OA[b] (Red) YM (Blue) GA, ISSA | Gray | Gray (Red) GA |
| Melanoid pigment | | | | |
| Peptone-yeast iron agar | – | – | – | – |
| Tyrosine agar | – | – | – | – |
| Tryptone-yeast liquid agar | – | – | – | – |
| Colour of substrate mycelium | +[c] Reddish-brown (ISSA) | + Yellow, Yellow-brown | + Orange-yellow | + Orange-yellow Dark reddish-orange |
| Soluble pigment | – | + Violet | + Red, yellow | + Yellow-orange, Pinkish red |
| Utilization of carbon source | | | | |
| Glucose | +[d] | + | + | + |
| Arabinose | –[d] | + | + | + |
| Xylose | – | + | + | + |
| Inositol | – | + | – | + |
| Mannitol | – | + | + | + |
| Fructose | – | + | + | + |
| Rhamnose | – | + | + | + |
| Sucrose | – | – | – | – |
| Raffinose | – | – | + | ± |
| Galactose | + | | | |

TABLE 3 (cont.)

| *St. recifensis* | *St. chibaensis* | *St. carnosus* | *St. corchorusii* | *St. viridifaciens* |
|---|---|---|---|---|
| RA, RF | S, RF | RA, S | S, RF, RA | RA, S, RF |
| Smooth | Smooth | Smooth | Smooth | Smooth |
| Gray | Gray (Yellow) GA | Gray (Red) YM, OA | Gray | Gray |
| –<br>–<br>– | –<br>–<br>– | –<br>–<br>– | –<br>–<br>– | –<br>–<br>– |
| — | — | — | — | — |
| – | – | + Pale pink or yellow (OA, GA) | – | + Yellowish green |
| + | + | + | + | + |
| + | + | ± | + | + |
| + | + | ± | + | + |
| – | + | ± | + | – |
| + | + | ± | + | – |
| + | + | ± | + | + |
| – | + | + | + | – |
| + | + | ± | + | + |
| + | + | – | + | – |
| | | | | + |

a)RA: Retinaculi-aperti, S: Spirales, RF: Rectiflexibiles.
b)ISSA: Inorganic salts-starch agar, OA: oatmeal agar, YM: yeast-malt extract agar, GA: Glycerin-asparagine agar.
c)+ Produced distinctive color
– Not produced distinctive color.
d)+ Good utilization
± Doubtful or variable utilization
– Poor or no utilization.

From these results mentioned above, strain AJ-9406 should belong to the new species of the genus *Steptomyces* and was named *St. mozunensis.*

Brief description of figures:

Figure 1 shows an infrared spectrum of the novel tryptophan derivative (ammonium salt) in KBr Disk.

Axis of Ordinate: transmittance (%)

Axis of Abscissa: wave number ($cm^{-1}$).

Utility in industry

The novel tryptophan derivative has an inhibitory activity against a metallo proteinase, and

therefore is useful for medicine such as an agent for treating *Pseudomonas aeruginosa* infectious disease and an agent for regulating growth of a microorganism.

**Claims**

1. N-(6-Deoxy-L-talosyloxyhydroxyphosphinyl)-L-leucyl-L-tryptophan, in which one or more hydroxy group in the sugar moiety is optionally protected by acylation, or a salt of the optionally protected compound, or an ester involving one or both acid moieties of the optionally protected compound.

2. A process for producing N-(6-deoxy-L-talosyloxyhydroxyphosphinyl)-L-leucyl-L-tryptophan, which process comprises culturing a microorganism belonging to the species *Streptomyces mozunensis* and capable of producing the tryptophan derivative, producing the tryptophan derivative in the culture medium and recovering it.

3. A process as set forth in claim 2 wherein the microorganism is *Streptomyces mozunensis* FERM-P 4589 and NRRL 12054.

4. *Streptomyces mozunensis* FERM-P 4589 and NRRL 12054 which is a new strain of the genus *Streptomyces*.

**Patentansprüche**

1. N-(6-Desoxy-L-talosyloxyhydroxyphosphinyl)-L-leucyl-L-tryptophan, in dem eine oder mehrere Hydroxygruppen in der Zuckereinheit gegebenenfalls durch Acylierung geschützt sind, Salz der gegebenenfalls geschützten Verbindung oder Ester, in dem eine oder beide Säuregruppen der gegebenenfalls geschützten Verbindung verestert sind.

2. Verfahren zur Herstellung von N-(6-Desoxy-L-talosyloxyhydroxyphosphinyl)-L-leucyl-L-tryptophan, bei dem ein Mikroorganismus der Spezies Streptomyces mozunensis, der zur Bildung des Tryptophanderivats befähig ist, gezüchtet wird, das Tryptophanderivat im Kulturmedium gebildet und gewonnen wird.

3. Verfahren nach Anspruch 2, bei dem der Mikroorganismus Streptomyces mozunensis FERM-P 4589 und NRRL 12054 ist.

4. Neuer Stamm des Genus Streptomyces, Streptomyces mozunensis FERM-P 4589 und NRRL 12054.

**Revendications**

1. N-(6-désoxy-L-taloxyloxyhydroxyphosphinyl)-L-leucyl-L-tryptophane, dans lequel un ou plusieurs des groupes hydroxy du reste de sucre sont facultativement protégés par acylation, ou un sel du composé faculitativement protégé ou un ester faisant intervenir l'un des deux ou les deux restes acides du composé facultativement protégé.

2. Un procédé pour produire le N-(6-désoxy-L-taloxyloxyhydroxyphosphinyl)-L-leucyl-L-tryptophane, procédé qui consiste à cultiver un micro-organisme appartenant à l'espèce *Streptomyces mozunensis* et capable de produire le dérivé de tryptophane, à produire le dérivé de tryptophane dans le milieu de culture et à le récupérer.

3. Un procédé selon la revendication 2, dans lequel le micro-organisme est *Steptomyces mozunensis* FERM-P 4589 et NRRL 12054.

4. *Streptomyces mozunensis* FERM-P 4589 et NRRL 12054 qui est une nouvelle souche du genre *Streptomyces*.

Figure 1

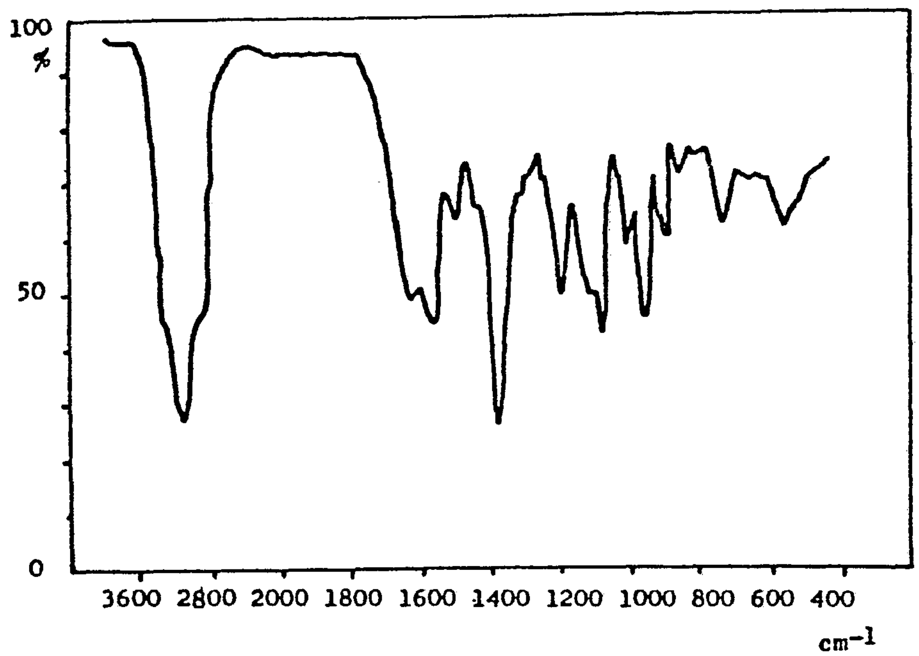
100
%
50
0
3600 2800 2000 1800 1600 1400 1200 1000 800 600 400
cm-1